# EUROPEAN PATENT APPLICATION

(11) **EP 4 474 821 A1**
(43) Date of publication of application: **11.12.2024**
(21) Application number: 23305889.0
(22) Date of filing: 05.06.2023
(51) Int. Cl.: G01N 33/50, G01N 33/68

(54) **METHODS FOR DIAGNOSING OR MONITORING MUSCULAR GLYCOGENOSIS**

(71) Applicant: GENETHON, 91000 Evry-Courcouronnes (FR); Institut National de la Santé et de la Recherche Médicale, 75013 Paris (FR); Université d'Evry Val d' Essonne, 91000 Evry-Courcouronnes (FR)
(72) Inventor: ROUILLON, Jérémy, 77310 Saint-Fargeau-Ponthierry (FR)
(74) Representative: Cabinet Becker et Associés

(57) **Abstract**

The invention relates to a method for prognosing, diagnosing, determining the risk, and monitoring the evolution of a muscular glycogenosis. It also relates to a method for evaluating the efficacy of a treatment of a muscular glycogenosis in a subject in need thereof.

## Description

### Field of the invention

The invention relates to a method for prognosing, diagnosing, determining the risk, and monitoring the evolution of muscular glycogenosis. It also relates to a method for evaluating the efficacy of a treatment of a muscular glycogenosis in a subject in need thereof.

### Background of the invention

Glycogenosis or glycogen storage diseases (GSDs) are metabolic disorders of glycogen metabolism. GSDs are caused by a lack of one enzyme involved in the synthesis or degradation of glycogen and are characterized by deposits of glycogen of normal or abnormal structure in tissues. Their prevalence is very low and they are considered rare diseases. There are several types of GSDs, which are classified by enzyme deficiency and affected tissue. Most of them are inherited in an autosomal recessive manner. Disorders of glycogen degradation may primarily affect the liver (hepatic glycogenosis), the muscles (muscular glycogenosis), or both (such as Pompe disease or GSDIII).

Efforts to establish clinical biomarkers associated with GSDs were very limited. The direct measurement of muscle glycogen for diagnosis and monitoring requires invasive biopsies and has limited sensitivity for late-onset forms of the disease because of the heterogeneity in the distribution of glycogen deposition. The only validated biomarker associated with muscular glycogenosis (such as GSDIII) is the elevation of creatine kinase (CK). However, serum CK levels can vary from day to day in the same patient, making them unreliable indicators to follow the efficacy of a treatment. In particular, serum CK would correlate to the degradation of muscle tissue under physical efforts rather than to the pathological state of the patient. In animal models, this biomarker is increased only in the plasma of old animals (9-12 month-old GSDIII rats and mice) and present significant fluctuations over time.

There is thus a need for new biomarkers to improve the diagnosis and prognosis of muscular glycogenosis.

### Summary of the invention

The present inventors have shown that the presence of myomesin protein, or of a fragment thereof, in particular of myomesin 3 or of a fragment thereof, in a biological fluid of a subject is associated to a muscular glycogenosis, such as GSDIII or GSDII (Pompe disease). The presence of myomesin protein or of a fragment thereof, in particular myomesin 3 or of a fragment thereof, in a biological fluid of a patient with a muscular glycogenosis has never been reported in the prior art. In addition, the results presented herein show that level of myomesin 3 correlate with the restoration of the muscle phenotype after gene therapy treatment of muscular glycogenosis.

Accordingly, an object of the present invention is the provision of a novel biomarker associated with the development of a muscular glycogenosis, or with the risk of having or developing a muscular glycogenosis. It is herein described the use of myomesin protein, in particular serum or plasmatic myomesin protein, more particularly myomesin 3, or of a fragment thereof as a biomarker of muscular glycogenosis.

The invention more particularly relates to a method for diagnosing, prognosing, monitoring the evolution or determining the risk of having or developing a muscular glycogenosis, comprising detecting the presence or absence of myomesin protein, or of a fragment thereof in a biological fluid sample of a subject. The presence of myomesin protein, in particular of myomesin 3, or of a fragment thereof being indicative of a muscular glycogenosis or of a risk of having or of developing a muscular glycogenosis.

In a particular embodiment, the method comprises :
a) measuring the level of said myomesin protein or of said fragment thereof in a biological fluid sample of the subject; and
b) comparing said level to the level of myomesin protein or of said fragment thereof in another biological fluid sample collected from said subject or from another subject, and/or to a reference level of said myomesin or of said fragment thereof.

In a particular embodiment, the method is for diagnosing a muscular glycogenosis in a subject, comprising :
a) measuring the level of myomesin protein or of a fragment thereof in a biological fluid sample of the subject; and
b) comparing said level to a reference level of said myomesin protein or of said fragment thereof;
wherein a higher level of myomesin protein or of said fragment thereof in a biological sample of the subject when compared to the reference level is indicative of a muscular glycogenosis or of a risk of having or of developing a muscular glycogenosis.

In a particular embodiment, said method is for prognosing or for monitoring the evolution of a muscular glycogenosis in a subject in need thereof, comprising:
a) measuring the level of myomesin protein or of a fragment thereof in a biological fluid sample of the subject,
b) comparing said level to the level of said myomesin protein or of said fragment in a biological fluid sample previously collected in the same subject;
the evolution of the level of said myomesin protein or of said fragment thereof being indicative of the progression of the disease.

In another particular embodiment, said method is for determining the efficacy of a treatment of a muscular glycogenosis in a subject in need thereof, comprising:
a) measuring the level of myomesin protein or of a fragment thereof in a biological fluid sample of the subject,
b) comparing said level to the level of said myomesin protein or of said fragment in a biological fluid sample previously collected in the same subject;
the evolution of the level of said myomesin protein or of a said fragment thereof being indicative of the progression of the disease and of the efficacy of the treatment.

In a particular embodiment, said method comprises:
a) measuring the level of said myomesin protein or of a fragment thereof in a biological fluid sample of the subject, whereby a reference level is determined; then
b) measuring the level of said myomesin protein or of said fragment in a second biological fluid sample collected in the same subject at a time after administration of the treatment, whereby a test level is determined; and
c) comparing the reference and test levels, the evolution of the level of said myomesin protein or of a fragment thereof being indicative of the progression of the disease and of the efficacy of the treatment.

In particular, a reduction of the level of said myomesin protein or of said fragment is indicative of an efficient treatment of the muscular glycogenosis.

According to a particular embodiment, the myomesin protein is myomesin 1, myomesin 2 or myomesin 3, in particular myomesin 3 or the fragment of the myomesin protein is a fragment of myomesin 1, myomesin 2 or myomesin 3, in particular a fragment of myomesin 3. In a particular embodiment, said myomesin protein is human myomesin 3 of SEQ ID NO:7 or said fragment is a fragment of human myomesin 3 of SEQ ID NO:7. In a particular embodiment, the fragment of myomesin 3 is a C-terminal fragment.
According to a particular embodiment, the biological fluid is blood, serum, plasma, saliva or urine. Preferably, the biological fluid is blood, serum or plasma. In a preferred embodiment, the biological fluid is plasma or serum, preferably plasma.

According to a particular embodiment, the method comprises detecting the presence or absence of myomesin protein or of a fragment thereof (such as myomesin 3 or a fragment thereof) by immunoassay such as enzyme-linked immunosorbent assay (ELISA), lateral flow immunoassay or western-blotting ; by capillary electrophoresis ; or by mass spectrometry. Preferably, the method comprises detecting the presence or absence of myomesin protein or of a fragment thereof (such as myomesin 3 or a fragment thereof) by immunoassay.

According to a particular embodiment, the muscular glycogenosis is selected from the group consisting of GSD 0 (muscle glycogen synthase deficiency), GSDII (Pompe disease), GSDIII (glycogen debranching enzyme deficiency), GSDIV (glycogen branching enzyme deficiency), GSDV (myophosphorylase deficiency), GSDVII (phosphofructokinase deficiency), GSDIXd (muscle phosphorylase kinase deficiency), GSDX (muscle phosphoglycerate mutase deficiency), GSDXI (lactate dehydrogenase deficiency), GSDXII (aldolase A deficiency), GSDXIII (β-enolase deficiency), GSDXIV (phosphoglucomutase deficiency, also known as CDG It), and GSDXV (muscle glycogenin deficiency), the muscular glycogenosis being preferably GSDII (Pompe disease) or GSDIII, the muscular glycogenosis being more preferably GSDIII. According to a particular embodiment, the muscular glycogenosis is GSDII (Pompe disease) or GSDIII. According to a preferred embodiment, the muscular glycogenosis is GSDIII.

According to a preferred embodiment, the myomesin protein is myomesin 3 or the fragment is a fragment of myomesin 3, and the muscular glycogenosis is GSDII (Pompe disease) or GSDIII. According to a particular embodiment, the myomesin protein is myomesin 3 or the fragment is a fragment of myomesin 3, and the muscular glycogenosis is GSDIII.
Another object of the invention relates to the use of myomesin protein, in particular of myomesin 3, or of a fragment of myomesin, in particular a fragment of myomesin 3 as a biomarker for diagnosing, prognosing, for monitoring the evolution, or for determining the risk of having or of developing a muscular glycogenosis, or for determining the efficacy of a treatment of a muscular glycogenosis. According to a preferred embodiment, the biomarker is myomesin 3 or a fragment of myomesin 3, and the muscular glycogenosis is GSDII (Pompe disease) or GSDIII. According to a particular embodiment, the biomarker is myomesin 3 or a fragment of myomesin 3, and the muscular glycogenosis is GSDIII.

Another object of the invention relates to a kit for implementing a method described above, comprising the means for detecting myomesin protein, in particular myomesin 3, or a fragment thereof, in particular a fragment of myomesin 3, and instructions for carrying out said method.

### Detailed description of the invention

The present invention provides a novel biomarker for diagnosis of muscular glycogenosis. The level of the marker in biological fluids, in particular in serum and plasma, according to the present invention is low in a normal state and high in the case of muscular glycogenosis, such as GSDII (Pompe disease) or GSDIII. Accordingly, the marker is useful for diagnosis intended to determine whether or not a patient carries muscular glycogenosis, prediction of the development of muscular glycogenosis, screening for a therapeutic agent or technique for muscular glycogenosis treatment, evaluation of the efficacy of a therapeutic agent or technique for muscular glycogenosis treatment, and other purposes.

According to the present invention, serum myomesin protein, and in particular myomesin 3, is associated to muscular glycogenosis, being at very low levels or absent in healthy subjects, but highly present in serum of patients in case of muscular glycogenosis, such as GSDII (Pompe disease) or GSDIII.

### Muscular glycogenosis

In the context of the present invention "muscular glycogenosis", "muscular glycogen storage disease", "muscular GSD", "muscle glycogenosis", "muscle glycogen storage disease" or "muscle GSD", refer to a glycogenosis affecting the muscles. In other words, "muscular glycogenosis" are muscular disorders caused by defective glycogen metabolism. "Muscular glycogenosis" affects at least the muscles, i.e. they can also affect other organs such as the liver or the brain.

Glycogen storage diseases (GSD) are inherited metabolic disorders of glycogen metabolism. They are divided into types 0 to XV, according to enzyme or transporter deficiency and organ distribution. Disorders of glycogen degradation mainly affect the liver, the muscle, or both. In muscle GSDs, the consequence of a block in skeletal muscle glycogenolysis or in glycolysis is an impairment of muscular performance, owing to an accumulation of glycogen that disrupts myofibrils and thus contractile function and/or to a inhibition of skeletal muscle energy (ATP) production. Generally, GSD are caused by the deficiency of enzymes involved in the metabolism of muscle glycogen. They can cause : excessive accumulation of glycogen molecules with normal structure ; excessive accumulation of glycogen molecules with abnormal structure ; or glycogen depletion, that is, the absence of glycogen synthesis.

Thus, in the context of the present invention, the muscular disease is due to the impairment of muscle function caused by defective glycogen metabolism in the muscles rather than by the destruction of muscle fibers itself, in contrast to what is observed in muscular dystrophies. In GSDs, the global muscle architecture is largely preserved, without cycles of necrosis and regeneration of muscle fibers. Neonatal forms are most often characterized by a decrease in muscle tone (or generalized hypotonia). Without early treatment, the evolution is rapid and fatal. Later forms evolve slowly. Some people develop permanent muscle weakness, particularly in the muscles of the shoulders, hips, arms and legs, whereas in some other muscular glycogenosis, the symptoms mostly occur transiently, often during intense exercise.

In a particular embodiment, the muscular glycogenosis is selected from the group consisting of GSD 0 (muscle glycogen synthase deficiency), GSDII (Pompe disease), GSDIII (glycogen debranching enzyme deficiency), GSDIV (glycogen branching enzyme deficiency), GSDV (myophosphorylase deficiency), GSDVII (phosphofructokinase deficiency), GSDIXd (muscle phosphorylase kinase deficiency), GSDX (muscle phosphoglycerate mutase deficiency), GSDXI (lactate dehydrogenase deficiency), GSDXII (aldolase A deficiency), GSDXIII (β-enolase deficiency), GSDXIV (phosphoglucomutase deficiency, also known as CDG It), and GSDXV (muscle glycogenin deficiency).

In a preferred embodiment, the muscular glycogenosis is selected from the group consisting of GSDII (Pompe disease), GSDIII, and GSDV.

In a preferred embodiment, the muscular glycogenosis is GSDII (Pompe disease) or GSDIII. In a preferred embodiment, the muscular glycogenosis is GSDIII.

Glycogen storage disease type 0 (GSD 0) is a disease characterized by a deficiency in the glycogen synthase enzyme. It is an autosomal recessive disorder of glycogen metabolism. In a particular embodiment, the glycogen storage disease is GSD type 0b (GSD-0b), which is caused by mutations in the *GYS1* gene, which encodes glycogen synthase in muscle and heart. It has been reported that patients showed muscle fatigability, hypertrophic cardiomyopathy, and sudden death.

Glycogen storage disease type 2 (GSDII), also known as Pompe disease, is an autosomal recessive lysosomal storage disease caused by a deficiency of acid alpha-glucosidase due to mutations in the *GAA* gene. This deficiency results in glycogen accumulation in the lysosomes in muscular tissues, leading to lysosomal swelling, cellular damage and organ dysfunction. This disease is either classified as early (infantile, classic) or late-onset (non-classic). Patients with infantile-onset Pompe disease develop severe hypotonia, respiratory insufficiency and left ventricular outflow obstruction. A common cause of lethality is respiratory insufficiency and left ventricular outflow obstruction. Patients with late-onset Pompe disease present with progressive muscular weakness and may develop respiratory insufficiency, although at an older age, which is an important cause of lethality.

Glycogen storage disease type 3 (GSDIII), also known as Cori disease or Forbes disease, is an autosomal recessive disorder of glycogen metabolism. GSDIII is caused by mutations in the *AGL* gene leading to genetic deficiency of glycogen debranching enzyme (GDE), an enzyme involved in glycogen degradation. Genetic deficiency of GDE blocks glycogenolysis in glycogen storage disease III (GSD III), resulting in accumulation of abnormal glycogen with short outer chain (limit dextrin) in various organs, mostly liver and muscle. In a particular embodiment, the glycogen storage disease is GSD type IIIa, in which GDE is deficient in both the muscle, the heart and the liver. Muscle weakness could be present during childhood. It becomes more prevalent in adults with onset of muscle weakness in the second or third decade, and patients in later stages can become wheel chair bound. Patients can also develop cardiomyopathy. There is significant clinical variability in the severity of the symptoms that these patients develop. The progressive myopathy and/or cardiomyopathy are major causes of morbidity in adults. There is still a need for a long-term treatment of GSDIII. Gene therapy aiming to stably replace the GDE protein in the affected tissues appears as a therapeutic approach.

Glycogen storage disease type 4 (GSDIV) is an autosomal recessive disorder of glycogen metabolism. GSDIV is caused by mutations in the *GBE1* gene, which encodes the glycogen branching enzyme. Therefore, glycogen is not made properly and abnormal glycogen molecules (amylopectin-like) accumulate in cells, such as cardiac and muscle cells. The liver, muscles, heart, nervous system, and other bodily tissues can be affected in GSDIV. In most affected individuals, symptoms and findings become evident in the first months of life, but onset during childhood or in adulthood have been reported. Patients present with muscle involvement including muscle weakness and cramps, as well as cardiomyopathy. Frequent liver and central nervous system impairment are frequent.

Glycogen storage disease type 5 (GSDV), also known as McArdle disease, is an autosomal recessive disorder of glycogen metabolism. GSDV is caused by mutations in the *PYGM gene,* which encodes muscle glycogen phosphorylase in muscle cells (myophosphorylase). *PYGM* gene mutations prevent the myophosphorylase from breaking down glycogen effectively. Deficiency of myophosphorylase leads to accumulation of muscle glycogen and a lack of glucose-1-phosphate. As a result, muscle cells cannot produce enough energy, so muscles become easily fatigued. Reduced energy production in muscle cells leads to the major features of GSD V. The number and severity of symptoms experienced may differ among people with this disease. This disease is characterized by exercise intolerance, muscle cramps, muscle pain, muscle stiffness, muscle weakness, and myoglobinuria, mostly during intense exercise.

Glycogen storage disease type 7 (GSDVII), also known as Tarui disease, is an autosomal recessive disorder of glycogen metabolism. GSDVII is caused by mutations in the *PFKM* gene, which encodes muscle phosphofructokinase. The severity of the condition and the associated signs and symptoms vary, but may include muscle weakness and stiffness; painful muscle cramps; nausea and vomiting; and/or myoglobinuria following moderate to strenuous exercise. Those who develop the condition during infancy may experience additional symptoms such as hypotonia, hypertrophic cardiomyopathy, and breathing difficulties. There are different types of GSDVII. They are differentiated by their signs and symptoms and the age at which symptoms first appear.

Glycogen storage disease type 9 (GSDIX), also known as phosphorylase kinase deficiency, is a disorder of glycogen metabolism. The phosphorylase kinase is a heterotetramer that is composed of four copies each of alpha, beta, gamma, and delta (calmodulin) subunits. Liver and muscle phosphorylase kinases exhibit tissue-specific subunit isoforms. In a particular embodiment, the glycogen storage disease is GSD type IXd, also known as X-linked muscle glycogenosis, which is caused by mutation in the *PHKA1* gene, which encodes the alpha subunit of muscle phosphorylase kinase. The typical clinical signs of GSDIXd are exercise intolerance, often combined with cramps, myalgia, and muscle weakness.

Glycogen storage disease type 10 (GSDX) is an autosomal recessive disorder of glycogen metabolism. GSDX is caused by mutations in the *PGAM2* gene, which encodes the muscle phosphoglycerate mutase. The typical presentations are exercise intolerance, cramps, and myoglobinuria during exercise.

Glycogen storage disease type 11 (GSDXI) is an autosomal recessive disorder of glycogen metabolism. GSDXI is caused by mutations in the *LDHA* gene, involved in glycolysis. Patients experienced exercise intolerance with recurrent rhabdomyolysis during intense effort.

Glycogen storage disease type 12 (GSDXII) is an autosomal recessive disorder of glycogen metabolism, caused by mutations in the *ALDOA* gene. Very few patients with this disease have been reported, mostly with muscle involvement and hemolytic anemia.

Glycogen storage disease type 13 (GSDXIII) is an autosomal recessive disorder of glycogen metabolism. GSDXIII is caused by mutations in the *ENO3* gene, which encodes the muscle beta-enolase. The typical presentations are exercise intolerance and myalgias.

Glycogen storage disease type 14 (GSDXIV or congenital disorder of glycosylation type It). It is an autosomal recessive disorder of both glycogen metabolism and protein glycosylation. GSDXIV is caused by mutations in the *PGM1* gene, which encodes the phosphoglucomutase. Very few patients have been reported, most of them presenting with muscle involvement (exercise intolerance, cramps, rhabdomyolysis during efforts and cardiomyopathy). Hypoglycemia can be present.

Glycogen storage disease type 15 (GSDXV) is an autosomal recessive disorder of glycogen metabolism. GSDXV is caused by mutations in the *GYG1* gene, which encodes the glycogenin. The typical presentations are muscle weakness, cardiomyopathy and sudden death.

### Subject

According to the invention, the terms "subject", "individual", and "patient" are used interchangeably herein and refer to a mammal that may be healthy (without any symptoms of muscular glycogenosis), thought to develop a muscular glycogenosis, suspected of suffering from muscular glycogenosis or suffering from muscular glycogenosis. Said subject for example presents at least one of the following symptoms: muscle cramping, muscle weakness, exercise intolerance, exercise-induced muscle cramps and weakness, muscle atrophy, delayed motor development, short stature, or cardiomyopathy. Said subject may also have displayed clinical manifestations of a muscular glycogenosis in the past, has been treated, and is monitored for potential disease recurrence. Said subject may also seem to be healthy but is at risk of developing a muscular glycogenosis or because a member of his family is suffering or has suffered from the same disease. The subject may also present a predisposition to a muscular glycogenosis identified thanks to genome analysis. The subject may also suffer from an already established muscular glycogenosis. The method of diagnosis of the present invention may also be applied to a subject with no known symptom or predisposition.

In one embodiment, the subject is a mammal, such as a human, primate, canine, murine, feline, bovine, ovine, swine or caprine subject. In a particular embodiment, the subject is a rodent subject (mouse or rat) or a human. In a particular embodiment, the subject is a human.

In some embodiments, the method is applied for mass screening of young children or of newborns. The present invention may also be implemented for detection of myomesin in heterozygous females, for predicting the risk of given birth to a child with a muscular glycogenosis. The invention may also be used for monitoring animal models, in particular canine models, mouse or rats models during preclinical evaluation of treatments. Mouse model include mouse with knock-down of the *Agl* or *Gaa* gene or expression of a non-functional GDE or GAA protein originating from mutation in the *Agl* or *Gaa* gene, for example *Agl*^{*-*/*-*} mice (for GSDIII) or *Gaa^{-l-}* mice (for GSDII). Rat model include rat with knock-down of the *Agl* or *Gaa* gene or expression of a non-functional GDE or GAA protein originating from mutation in the *Agl* or *Gaa* gene, for example *Agl*^{*-*/*-*} rats (for GSDIII) or *Gaa^{-l-}* rats (for GSDII).

In a particular embodiment the subject is an adult, an adolescent or a child, in particular a child being of 10 or less than 10 year-old, in particular less than 5 year-old, in particular less than 4 year-old, more particularly a child being 3 year-old or less than 3 year-old. In one embodiment, the subject is a 3- to 10-year old human subject. In an embodiment, the subject is a newborn. Accordingly, the detection of a muscular glycogenosis or of a risk of having or of developing a muscular glycogenosis may be done at an early stage and even before symptoms of the diseases are observed. This marker is also useful in young subjects for predicting the development of muscular glycogenosis, screening for a therapeutic agent or technique for muscular glycogenosis, evaluation of the efficacy of a therapeutic agent or technique for muscular glycogenosis, and other purposes.

### Sample

The methods of the invention require a biological fluid sample from the subject.

The terms "biological fluid" include body effluents and excretions such as blood, serum, plasma, saliva, urine or feces. In a particular embodiment, the biological fluid sample is a blood, serum or plasma sample. According to a particular embodiment of the invention, the biological fluid sample is a serum or plasma sample.

According to the present invention, a "reference sample" may correspond to a biological fluid sample obtained from one or more subjects, preferably two or more, who do not have a muscular glycogenosis. The "reference sample" may also correspond to a sample obtained from one or more patients having a muscular glycogenosis. In the context of a method for prognosing a patient, for monitoring the evolution of the disease in a patient or for evaluating the efficacy of a treatment of a muscular glycogenosis in a patient, the reference sample may in particular be a sample previously collected from said patient, in particular previous to a treatment, or in an earlier time of the treatment.

### Myomesin

The myomesin family of proteins comprises closely related structural proteins detected at the M-band of the sarcomere in striated muscles: myomesin-1 (in *homo sapiens, UniprotKB* / Swiss-Prot accession number P52179 - SEQ ID NO: 1; in *mus musculus, UniprotKB* / Swiss-Prot accession number Q62234- SEQ ID NO:2; in *rattus norvegicus, UniprotKB* / Swiss-Prot accession number A0A0G2K5P5- SEQ ID NO:3), myomesin-2 (in *homo sapiens, UniprotKB* / Swiss-Prot accession number P54296 - SEQ ID NO:4; in *mus musculus, UniprotKB* / Swiss-Prot accession number Q3UQS9- SEQ ID NO:5; in *rattus norvegicus, UniprotKB* / Swiss-Prot accession number A0A8I6AC90- SEQ ID NO:6) and myomesin-3 (in *homo sapiens,* UniprotKB / Swiss-Prot accession number Q5VTT5 - SEQ ID NO:7; in *mus musculus, UniprotKB* / Swiss-Prot accession number A2ABU4- SEQ ID NO:8; in *rattus norvegicus, UniprotKB* / Swiss-Prot accession number D4A228- SEQ ID NO:9). These proteins are involved in sarcomere stability and resistance during intense or sustained stretching (Schoenauer et al., 2008, Myomesin 3, a novel structural component of the M-band in striated muscle. Journal of molecular biology 376: 338-351).

In a particular embodiment of the present invention, the detection of myomesin protein or of a fragment thereof includes the detection of myomesin-1, myomesin-2 or myomesin-3, or of a fragment of myomesin-1, myomesin-2 or myomesin-3.

In a particular embodiment of the invention, the detection of myomesin protein or of a fragment thereof includes the detection of human myomesin-1, human myomesin-2 or human myomesin-3, or of a fragment of human myomesin-1, human myomesin-2 or human myomesin-3. In a preferred embodiment of the invention, the detection of myomesin protein or of a fragment thereof includes the detection of human myomesin-3, or of a fragment of human myomesin-3.

In another particular embodiment of the invention, the detection of myomesin protein or of a fragment thereof includes the detection of mouse myomesin-1, mouse myomesin-2 or mouse myomesin-3, or of a fragment of mouse myomesin-1, mouse myomesin-2 or mouse myomesin-3. In a preferred embodiment of the invention, the detection of myomesin protein or of a fragment thereof includes the detection of mouse myomesin-3, or of a fragment of mouse myomesin-3. Such detection of mouse myomesin may be used for monitoring mouse models of GSD, during preclinical evaluation of treatments.

In another particular embodiment of the invention, the detection of myomesin protein or of a fragment thereof includes the detection of rat myomesin-1, rat myomesin-2 or rat myomesin-3, or of a fragment of rat myomesin-1, rat myomesin-2 or rat myomesin-3. In a preferred embodiment of the invention, the detection of myomesin protein or of a fragment thereof includes the detection of rat myomesin-3, or of a fragment of rat myomesin-3. Such detection of rat myomesin may be used for monitoring rat models of GSD, during preclinical evaluation of treatments.

In another particular embodiment of the invention, the detection of myomesin protein or of a fragment thereof includes the detection of myomesin-3, such as human, mouse or rat myomesin-3, or of a fragment of myomesin-3, such as a fragment of human, mouse or rat myomesin-3. In a particular embodiment of the invention, the detection of myomesin protein or of a fragment thereof includes the detection of human myomesin-3, or of a fragment of human myomesin-3.

There has been no report regarding any correlation between the myomesin or a fragment thereof being present in biological fluids and muscular glycogenosis.

In a particular embodiment, the "fragment of myomesin" is any fragment that is specific to myomesin (myomesin-1, myomesin-2 or myomesin-3, preferably myomesin-3). In other words, it can be any fragment that, when detected in the sample, confirm the presence of myomesin in the sample. In a particular embodiment, the fragment is of at least 6 amino acids, such as at least 7 amino acids, 8 amino acids, 9 amino acids or at least 10 amino acids.

In a preferred embodiment, the methods of the present invention comprise detecting myomesin-3 or a fragment thereof. The sequence of human myomesin 3 is known, and shown in SEQ ID NO:7. In some embodiments, the methods of the invention comprise detecting or quantifying a fragment of myomesin 3.

In a particular embodiment, the detected fragment is a C-terminal fragment of myomesin 3, in particular a fragment of about 110 or 140 kDa.

In some embodiments, the fragment detected is comprised between amino acids 350 and 1350 of SEQ ID NO:7 or of any natural variant of human myomesin 3, for example of corresponding fragments in a sequence presenting at least 90 %, preferably 95 %, more preferably 99 % identity with SEQ ID NO:7. In particular, the fragment detected is comprised between amino acids 355 and 1322 of SEQ ID NO:7 or of any natural variant of human myomesin 3, for example of corresponding fragments in a sequence presenting at least 90 %, preferably 95 %, more preferably 99 % identity with SEQ ID NO:7.

In a further particular embodiment, the invention implements the detection of a fragment of human myomesin 3 comprising amino acids 355-363 (SEQ ID NO:10), 364-380 (SEQ ID NO:11), 461-474 (SEQ ID NO:12), 539-556 (SEQ ID NO:13), 560-567 (SEQ ID NO:14), 577-596 (SEQ ID NO:15), 640-657 (SEQ ID NO:16), 695-713 (SEQ ID NO:17), 761-773 (SEQ ID NO:18), 776-795 (SEQ ID NO:19), 1008-1019 (SEQ ID NO:20), 1076-1086 (SEQ ID NO:21), 1130-1141 (SEQ ID NO:22), 1219-1228 (SEQ ID NO:23) and/or 1303-1322 (SEQ ID NO:24) of human myomesin 3 as represented in SEQ ID NO:7, or of any natural variant of myomesin 3, for example of corresponding fragments in a sequence presenting at least 90 %, preferably 95 %, more preferably 99 % identity with SEQ ID NO:7.

According to another embodiment, the fragment of myomesin 3 detected according to the invention is selected as a fragment of the whole protein migrating in SDS-PAGE gel at the positions corresponding approximately to 110 or 140 kD.

### Methods of detection

The presence or absence of myomesin (in particular myomesin 3) or of a fragment thereof may be detected by a number of techniques known in the art for the detection of protein such as:
- immunoassay, such as enzyme-linked immunosorbent assay (ELISA), lateral flow (immuno)assay or western-blotting,
- Capillary electrophoresis ; and
- mass spectrometry.

For example, expression of a marker protein in a sample can be determined by immunoassays (immunological assay techniques). Specifically, expression of a marker protein in the sample can be determined based on the reaction between such protein and an antibody that specifically binds thereto. Immunoassays may be carried out in a liquid phase or solid phase, provided that the technique used is conventional in the art. For ease of detection, use of a solid phase may be preferable. In addition, immunoassay techniques are not limited, and immunoassay can be carried out by sandwich assay, competitive assay, Western blotting, or ELISA, as well as a direct solid-phase assay. Any antibody that is able to specifically bind to myomesin, in particular myomesin 3, or to a fragment thereof, and more particularly to a C-terminal fragment of myomesin 3 may be used in immunoassays according to the invention. In some embodiments, the fragment detected is comprised between amino acids 350 and 1350 of SEQ ID NO:7 as provided above (the fragment being in particular a sequence comprising or consisting of one or more of SEQ ID NO: 10 to SEQ ID NO:24) or of any natural variant of myomesin 3. The antibody may be a monoclonal or polyclonal antibody. Alternatively, it may be, for example, a Fab or Fv fragment capable of binding to an epitope of a marker protein. When a primary antibody and a secondary antibody are used, both may be monoclonal antibodies. Alternatively, either the primary or secondary antibody may be a polyclonal antibody. An antibody can be prepared by a method known in the art, or a commercially available antibody may be used such as the anti-myomesin 3 antibody from Proteintech which is a polyclonal rabbit antibody (17692-1-AP, Proteintech) against the C-terminal 325 amino acids of myomesin 3 or a commercially available antibody may be used such as the anti-myomesin 2 from Santa Cruz (myomesin-2 antibody (H-65): sc-50435) against amino acids 913-977 mapping within an internal region of myomesin-2 of human origin.

Capillary electrophoresis (CE) is a family of electrokinetic separation methods performed in submillimeter diameter capillaries and in micro- and nanofluidic channels. Very often, CE refers to capillary zone electrophoresis (CZE), but other electrophoretic techniques including capillary gel electrophoresis (CGE), capillary isoelectric focusing (CIEF), capillary isotachophoresis and micellar electrokinetic chromatography (MEKC) belong also to this class of methods. In capillary electrophoresis methods, analytes migrate through electrolyte solutions under the influence of an electric field. Analytes can be separated according to ionic mobility and/or partitioning into an alternate phase via non-covalent interactions. Additionally, analytes may be concentrated or "focused" by means of gradients in conductivity and pH. The system's main components are a sample vial, source and destination vials, a capillary, electrodes, a high voltage power supply, a detector, and a data output and handling device. The source vial, destination vial and capillary are filled with an electrolyte such as an aqueous buffer solution. To introduce the sample, the capillary inlet is placed into a vial containing the sample. Sample is introduced into the capillary via capillary action, pressure, siphoning, or electrokinetically, and the capillary is then returned to the source vial. The migration of the analytes is initiated by an electric field that is applied between the source and destination vials and is supplied to the electrodes by the high-voltage power supply. In the most common mode of capillary electrophoresis, all ions, positive or negative, are pulled through the capillary in the same direction by electroosmotic flow. The analytes separate as they migrate due to their electrophoretic mobility, and are detected near the outlet end of the capillary. The output of the detector is sent to a data output and handling device such as an integrator or computer. The data is then displayed as an electropherogram, which reports detector response as a function of time. Separated compounds appear as peaks with different migration times in an electropherogram.

Alternatively, the expression of a marker protein can be determined by mass spectrometry (MS) and comprises the identification of proteins by measuring the ratio between mass and charge (m/z) of peptides obtained from their enzymatic digestion (MS) or of their fragments (MS/MS). The m/z of thus generated peptides are then compared to the calculated m/z of protein sequences present in databases permitting the protein identification. According to a particular embodiment, the multiple reaction monitoring (MRM) approach can be used in the present invention, in order to allow quantitative and/or qualitative detection of the peptides in biological fluids. The results are presented as a mass spectrum, a plot of intensity as a function of the mass-to-charge ratio. In a typical MS procedure, the fragments are separated according to their mass-to-charge ratio, for example by accelerating them and subjecting them to an electric or magnetic field: ions of the same mass-to-charge ratio will undergo the same amount of deflection. The ions are detected by a mechanism capable of detecting charged particles, such as an electron multiplier. A mass spectrometer consists of three components: an ion source, a mass analyzer, and a detector.

The presence or absence of myomesin (in particular myomesin 3) or of a fragment thereof may be detected by any technique known in the art for the detection of protein. Another example of suitable technique is the strategy of nanopore-based protein sequencing and sensing (as described in Alfaro, J.A., Bohländer, P., Dai, M. et al. The emerging landscape of single-molecule protein sequencing technologies. Nat Methods 18, 604-617 (2021)).

### Methods

In a particular embodiment, the method of the invention is for the diagnosis of a muscular glycogenosis, or for determining the risk of having or of developing a muscular glycogenosis, comprising detecting the presence or absence of myomesin, in particular myomesin 3 or of a fragment thereof in a biological fluid sample of a subject, the presence of myomesin (in particular myomesin 3), or of a fragment thereof being indicative of a muscular glycogenosis or of the risk of having or of developing a muscular glycogenosis.

In a particular embodiment, the method comprises :
a) measuring the level of said myomesin or of said fragment thereof in a biological fluid sample of the subject; and
b) comparing said level to the level of myomesin or of said fragment thereof in another biological fluid sample collected from said subject or from another subject, and/or to a reference level of said myomesin or of said fragment thereof.

Said "another" biological fluid sample is a distinct biological fluid sample collected from the same subject at another time point or collected from another subject, and is preferably the same type of biological fluid (such as serum) as the first biological sample collected.

According to a particular aspect, the method is for diagnosing a muscular glycogenosis in a subject, comprising :
a) measuring the level of myomesin or of a fragment thereof in a biological fluid sample of the subject; and
b) comparing said level to a reference level of said myomesin or of said fragment thereof ;
wherein a higher level of myomesin or of said fragment thereof in a biological sample of the subject when compared to the reference level is indicative of a muscular glycogenosis or of a risk of having or of developing a muscular glycogenosis. According to this embodiment, the "reference level" may correspond to the level of myomesin or a fragment thereof (in particular myomesin 3 or a fragment thereof) in a biological fluid sample obtained from one or more subjects, such as two or more, who do not have a muscular glycogenosis. For example, the "reference level" may be a reference value corresponding to the average level of myomesin or of a fragment thereof (in particular myomesin 3 or a fragment thereof), detected in biological fluid samples (such as blood, serum or plasma) in a panel of healthy subjects, who do no have a muscular glycogenosis.

According to another aspect, the invention relates to a method for monitoring a muscular glycogenosis. In this case, the method may comprise the step of quantifying the level of myomesin, in particular myomesin 3, or of fragments thereof at a T1 time in the subject, a level higher at a time T2 following T1 being indicative of a disease progression and a lower or equal expression at T2 being indicative of a remission or stabilization. The evolution of the level of myomesin or of a fragment thereof, in particular myomesin 3 or myomesin 3 fragment level may also be compared to a reference sample from a patient of an equivalent age having a muscular glycogenosis. In a particular embodiment, the method is for prognosing or for monitoring the evolution of a muscular glycogenosis in a subject in need thereof, comprising:
a) measuring the level of myomesin or of a fragment thereof in a biological fluid sample of the subj ect,
b) comparing said level to the level of said myomesin or of said fragment in a biological fluid sample previously collected in the same subject;
the evolution of the level of said myomesin or of said fragment thereof being indicative of the progression of the disease. In particular, an increase in the level of myomesin or of a fragment thereof being indicative of a disease progression and decrease in the level of myomesin or of a fragment thereof being indicative of a remission or stabilization.

According to a further aspect, the invention relates to a method for determining the efficacy of a treatment of muscular glycogenosis. In this case, the method may comprise the step of quantifying the level of the myomesin protein (e.g. myomesin 3) or of a fragment thereof at a T1 time in the subject, a level lower at a time T2 following T1 being indicative of an efficient treatment while an increased or equal expression at T2 being indicative of an inefficient treatment or less efficient treatment. The evolution of the myomesin (e.g. myomesin 3) or myomesin fragment (e.g. myomesin 3 fragment) level may also be compared to a reference sample from a patient of an equivalent age having a muscular glycogenosis. A lower level in the subject than in the reference sample is indicative of an efficient treatment of the muscular glycogenosis.

By "higher expression level" or "lower expression level" is meant an expression level the variation of which is significant, according to methods well known by those skilled in the art. In a particular embodiment a significant higher expression level corresponds to an increase in myomesin level of at least 10%, at least 20%, at least 30%, at least 40%, or at least 50%. In a particular embodiment a significant lower expression level corresponds to a decrease in myomesin level of at least 10%, at least 20%, at least 30%, at least 40%, or at least 50%.

Both methods of monitoring a muscular glycogenosis progression and of determining the efficacy of a treatment of muscular glycogenosis may involve the use of a quantitative method for determine the level of myomesin protein (e.g. myomesin 3) or of a fragment thereof, such as ELISA or MRM mass spectrometry. In addition, both methods include the collection of samples at different times (for example the collections are made several days/weeks/months away). In addition, in the method for determining the efficacy of a treatment, the first and second samples may be collected both after the treatment has been provided to the patient (for example, the first sample is collected after the treatment, the same day as the treatment or several days/weeks/months after the treatment, and the second sample is collected several days/weeks/months after collection of the first sample).

Differential expression of different myomesins in muscle fibres (MYOM3) was found mainly in intermediate speed fibres (type IIa) of skeletal muscle, while fast fibres express more MYOM2 and MYOM1 is expressed in all muscle fibres (Schoenauer et al., 2008, Myomesin 3, a novel structural component of the M-band in striated muscle. Journal of molecular biology 376: 338-351) implies a possibility to follow the results of therapeutic treatment for each type of muscle fibres. Accordingly, the invention also provides a method for following the outcome of a therapeutic treatment of a muscular glycogenosis, comprising detecting the absence or the presence, and/or the level of at least one myomesin protein or of at least one fragment thereof selected from myomesin 1, myomesin 2 and myomesin 3 in a biological fluid of a subject, in particular detecting the absence of presence of all three proteins, to thereby determine the impact of the treatment on each type of muscle fibres.

In another aspect, the detection of myomesin protein (e.g. myomesin 3) or of a fragment(s) thereof can be used to predict the clinical outcome of a subject afflicted with muscular glycogenosis, said method comprising: a) determining the level or amount of myomesin protein (e.g. myomesin 3) or of fragment(s) thereof, in a biological sample of said subject, and comparing same to a reference value, b) predicting the clinical outcome based on the comparison of step a).

It is further disclosed a method for identifying or screening a therapeutic agent or candidate therapeutic agent, for use in treating a muscular glycogenosis in a subject, which method comprises administering the subject with said therapeutic agent and detecting the presence of myomesin protein (e.g. myomesin 3) or of a fragment(s) thereof in a biological sample from the subject, wherein a decreased of level of myomesin protein of fragment thereof after administration of the therapeutic agent is indicative of an agent that has a benefit on the muscular glycogenosis in the subject. According to this particular embodiment, the subject can be a mammal, such as a human, primate, canine, murine, feline, bovine, ovine, swine or caprine subject. In a particular embodiment, the subject is a rodent subject (mouse or rat) or a human. In a preferred embodiment, the subject is a human.

Furthermore, the diagnostic, prognosis, monitoring, determination of the risk of having or of developing the disease or the determination of the efficacy of the treatment may be confirmed in a further step by methods known in the art for the evaluation of muscular glycogenosis, such as functional assessment of the muscle (clinical evaluation, 6-min walking test), the heart (echocardiography) and/or the respiratory function (blood gaz analysis, pulmonary function tests), such as quantification of CK levels in serum, such as muscle biopsy (showing among other glycogen accumulation, vacuolar myopathy, distortion of myofibril architecture), enzymatic testing when available in leukocytes or muscle biopsy and/or genetic testing.

### Kit

Another aspect of the invention relates to a kit for the diagnosis, prognosis, monitoring or determination of the risk of having or of developing a muscular glycogenosis or the determination of the efficacy of a treatment of a muscular glycogenosis, comprising means for detecting or dosing myomesin protein, in particular of myomesin 3, or a fragment thereof in a biological fluid sample from a subject. In a particular embodiment, the kit comprises means for implementing an ELISA or Western-blot analysis. For example the kit comprises a specific means for detecting myomesin protein, such as myomesin 3, or a fragment thereof, such as an antibody as described above. The kit may also comprise instructions for carrying out the method of the invention.

The present invention will now be described with reference to the following figures and examples.

### Legends of the figures

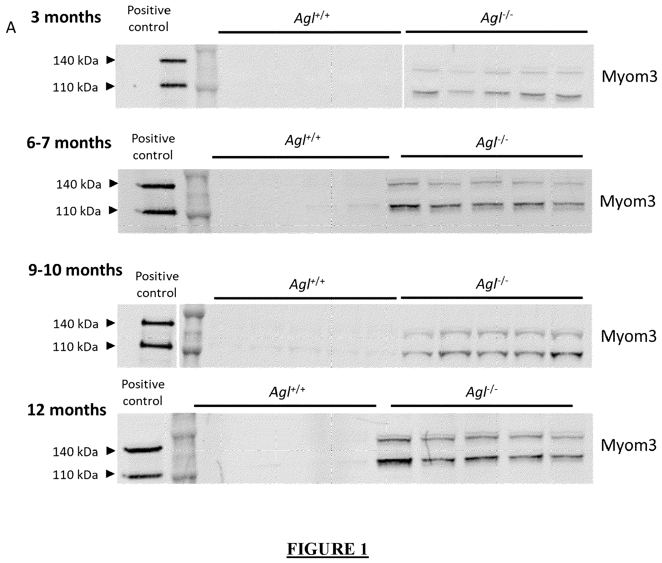

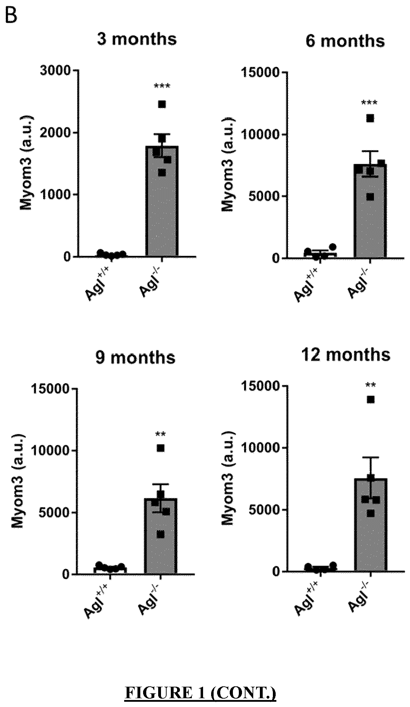

**Figure 1****. Levels of myomesin-3 fragments (MYOM3) in plasma of *Agl*^{+/+} and *Agl^{-l-}* mice.** (A) Western blot analysis of MYOM3 levels in plasma of *Agl*^{+/+} and *Agl*^{*-*/*-*} mice at 3, 6, 9 and 12 months of age. Serum from a *mdx* mice was used as positive control. (B) Quantification of the Western Blot in panel A (arbitrary unit). Statistical analysis performed by Student T test (***p<0.001 ; **p<0.01, *p<0.05).

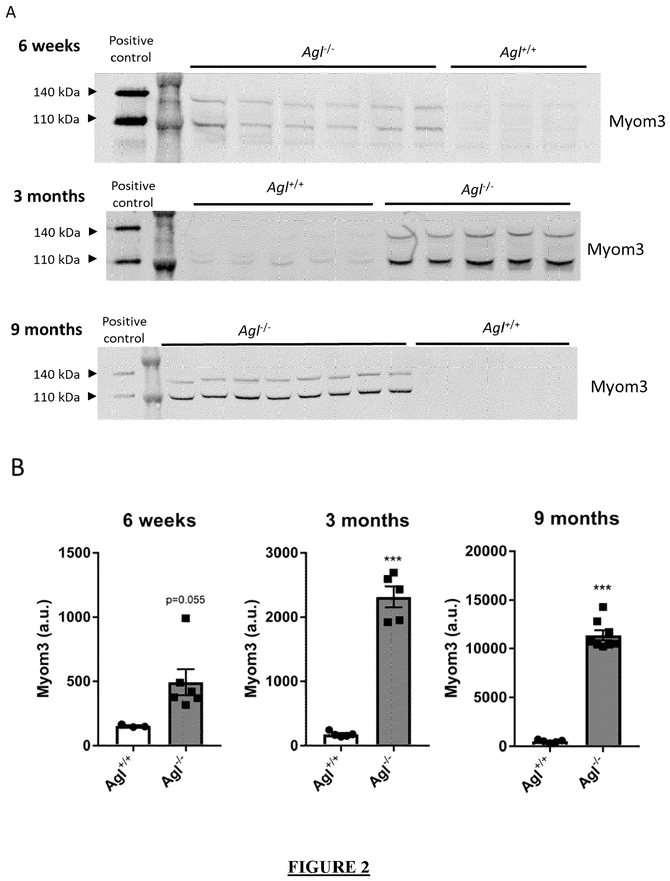

**Figure 2****. Levels of myomesin-3 fragments (MYOM3) in plasma of *Agl*^{+/+} and *Agl*^{*-*/*-*} rats.** (A) Western blot analysis of MYOM3 levels in plasma of *Agl*^{+/+} and *Agl*^{*-*/*-*} rats at 6 weeks, 3 months and 9 months of age. Plasma from a *mdx* mice was used as positive control. (B) Quantification of the Western Blot in panel A (arbitrary unit). Statistical analysis performed by Student T test (***p<0.001 ; **p<0.01, *p<0.05).

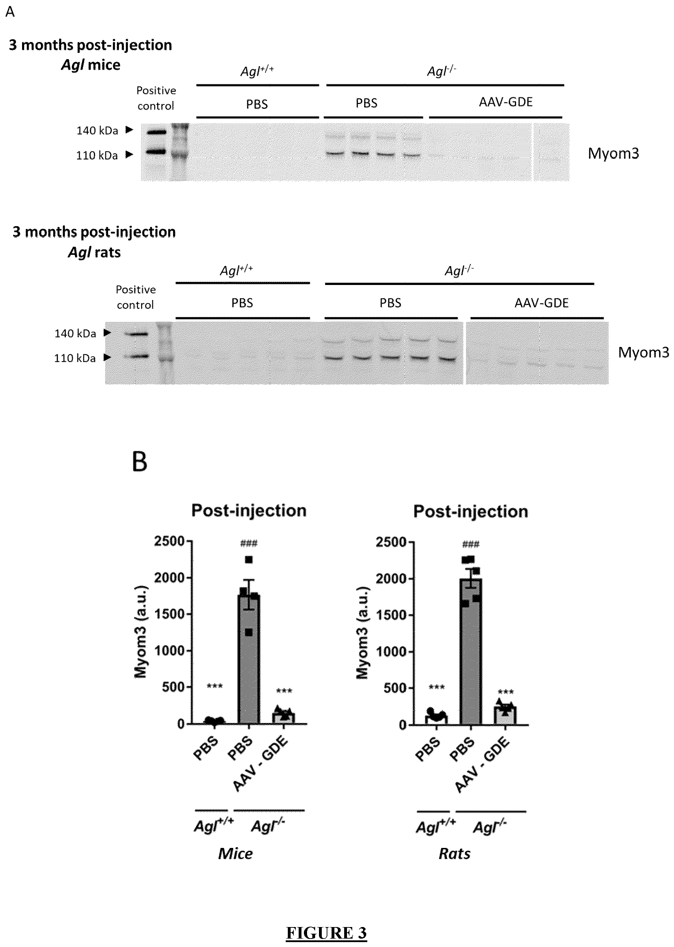

**Figure 3****. Levels of myomesin-3 fragments (MYOM3) in plasma of *Agl*^{+/+} and *Agl*^{*-*/*-*} mice and rats treated with an AAV vector encoding the missing GDE enzyme.** An AAV vector encoding a mini-GDE enzyme was administered intravenously to 3-month-old *Agl*^{-/-} mice or 6-week-old *Agl*^{*-*/*-*} rats. Of note, these animals displayed complete correction of the muscle phenotype 3 months after injection. Controls *Agl*^{+/+} and *Agl*^{*-*/*-*} animals were injected with phosphate buffer saline (PBS). (A) Western blot analysis of MYOM3 levels 3 months post-injection in *Agl* mice (upper panel) and *Agl* rats (lower panel). Plasma from a *mdx* mice was used as positive control. (B) Quantification (arbitrary unit) of the Western Blot in panel A for *Agl* mice (left panel) and rats (right panel). Statistical analysis performed by One-way ANOVA (***p<0.001 ; **p<0.01, *p<0.05 vs. *Agl*^{*-*/*-*} animals injected with PBS et ###p<0.001 ; ##p<0.01, #p<0.05 vs. *Agl*^{+/+} animals injected with PBS).

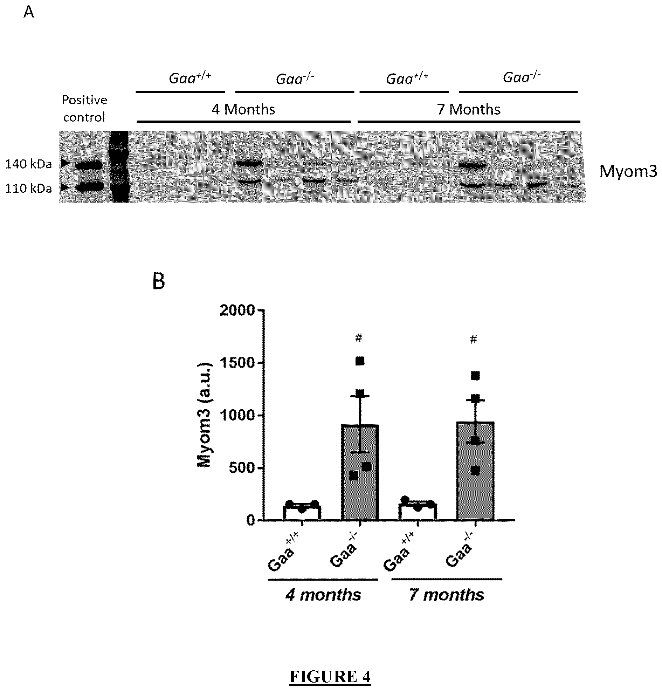

**Figure 4****. Levels of myomesin-3 fragments (MYOM3) in plasma of *Gaa*^{+/+} and *Gaa*^{*-*/*-*} mice.** (A) Western blot analysis of MYOM3 levels in plasma of *Gaa*^{+/+} and *Gaa*^{*-*/*-*} mice at 4 and 7 months of age. Plasma from a *mdx* mice was used as positive control. (B) Quantification of the Western Blot in panel A (arbitrary unit). Statistical analysis performed by One-way ANOVA (###p<0.001 ; ##p<0.01, #p<0.05 vs. *Agl*^{+/+} animals of corresponding age).

### Examples

### MATERIALS AND METHODS

### In Vivo Studies

The *Agl* knockout mice (*Agl*^{*-*/*-*}, *Agl*^{tm1b(EUCOMM)Wtsi}) were previously described [Vidal et al. Rescue of GSDIII Phenotype with Gene Transfer Requires Liver- and Muscle-Targeted GDE Expression. Mol Ther. 2018 Mar 7;26(3):890-901] and are of mixed background (C57BL/6J and Balb/c). The *Agl* knock-out rats were of Sprague Dawley background and were generated by our team. *Gaa* knockout mice (*Gaa*^{*-*/*-*}) were purchased from The Jackson Laboratory (B6;129*-Gaa*^{*tm1Rabn*/*J,*} stock number 004154, 6neo) and originally generated by *Raben et al.* [ Raben et al. Targeted disruption of the acid alpha-glucosidase gene in mice causes an illness with critical features of both infantile and adult human glycogen storage disease type II. J Biol Chem. 1998 Jul 24;273(30): 19086-92]. All animal studies were performed according to the French and European legislation on animal care and experimentation (2010/63/EU) and approved by the local institutional ethical committee.

### Production and administration of rAAV Vectors

All rAAV vectors used in this study were produced using an adenovirus-free transient transfection method and purified using a chromatographic method as described earlier [Collaud et al. Preclinical Development of an AAV8-hUGT1A1 Vector for the Treatment of Crigler-Najjar Syndrome. Mol Ther Methods Clin Dev. 2018 Dec 31;12:157-174]. AAV vectors encode a functional truncated version of GDE, able to be packaged in the expression cassette and previously shown to be able to correct the muscle phenotype.

Phosphate buffer saline (PBS) or rAAV vectors encoding GDE were administered intravenously via the tail vein to 3-month-old male *Agl*^{-/-} mice and wild-type littermates (*Agl*^{+/+}) or to 6-week-old male *Agl*^{*-*/*-*} rats and wild-type littermates (*Agl*^{+/+})*.* Blood sampling was performed under anesthesia using Isuflorane.

### Western Blot Analysis

50 µg of proteins from plasma samples were used for Western Blot. Serum of *mdx* mice were used as a positive control. SDS-PAGE electrophoresis was performed in a 4-12% Bis-Tris gradient polyacrylamide gel (NuPAGE^{™}, Invitrogen). After transfer, the membrane was blocked with Intercept Blocking buffer (LI-COR Biosciences) and incubated with an anti-myomesin3 (MYOM3) rabbit polyclonal antibody (17692-1-AP, Proteintech). The membrane was washed and incubated with an anti-rabbit secondary antibody (LI-COR Biosciences) and visualized by Odyssey imaging system (LI-COR Biosciences).

### Statistical Analysis

All the data shown are reported as mean ± SEM. The GraphPad Prism^{™} software was used for statistical analysis. p values < 0.05 were considered significant. For all the datasets, data were analyzed by parametric tests, alpha = 0.05 (Student T test or one-way ANOVA with Tukey's post hoc correction). The statistical analysis performed for each dataset is indicated in each figure legend.

### RESULTS

### Presence of MYOM3 fragments in plasma of Agl^{-/-} mice (model of GSDIII)

The presence of the MYOM3 fragments was analyzed in *Agl*^{+/+} mice and *Agl*^{*-*/*-*} mice (murine model for GSDIII), aged 3 months to 12 months. Western blot analysis with antibodies against the C-terminal part of MYOM3 showed the presence of two fragments of 110 kDa and 140 kDa in the plasma of *Agl*^{*-*/*-*} mice at all ages tested **(****Figure 1**). These fragments were undetectable in healthy controls (*Agl*^{+/+} mice). Of note, *Agl*^{*-*/*-*} mice exhibit muscle impairment as early as 3 months of age (glycogen accumulation and muscle weakness) [Vidal et al. Rescue of GSDIII Phenotype with Gene Transfer Requires Liver- and Muscle-Targeted GDE Expression. Mol Ther. 2018 Mar 7;26(3):890-901].

### Presence of MYOM3 fragments in plasma of Agl^{-/-} rats (model of GSDIII)

The presence of the MYOM3 fragments was also analyzed in *Agl*^{+/+} rats and *Agl*^{-/-} rats (model for GSDIII), aged from 6 weeks to 9 months by Western blot analysis. The fragments of MYOM3 were detected in *Agl*^{*-*/*-*} rats at all ages tested, even at 6-week **(****Figure 2****)** and were undetectable in the healthy controls (*Agl*^{+/+} rats). Interestingly, the level of MYOM3 in the plasma increased progressively (from 6 weeks to 9 months). Of note, *Agl*^{-/-} rats exhibit muscle involvement (glycogen increase) from 6 weeks of age.

The results thus show an elevation of MYOM3 in these two models of GSDIII at all ages evaluated.

### Levels of myomesin3 fragments (MYOM3) are normalized in plasma of Agl^{-/-} mice and rats treated with an AAV vector encoding the missing GDE enzyme.

Very interestingly, correction of the muscle phenotype by a gene therapy approach using an AAV vector encoding the missing GDE (Glycogen-debranching enzyme) allows normalization of this biomarker in both *Agl*^{-/-} mice and rats at 3 months post-injection **(****Figure 3****).** Of note, these animals displayed complete correction of the muscle phenotype (normalization of glycogen content and muscle strength in mice and normalization of glycogen content in rats) at this time point. Thus, the level of MYOM3 can be used to monitor the muscle recovery after a treatment in muscle glycogenosis, such as gene therapy. It is a simple measurement that can be performed on plasma and is therefore much less invasive than muscle biopsies.

### Presence of MYOM3 fragments in plasma of Gaa^{-/-} mice (model of Pompe disease)

Analysis of MYOM3 was performed by Western blot in plasma from a mice model for another type of glycogenosis, i.e. GSD II (Pompe disease). In particular, the presence of MYOM3 was analyzed in *Gaa*^{+/+} mice and *Gaa*^{-/-} mice (murine model for GSDII, Pompe disease), aged 4 months and 7 months. Western blot analysis with antibodies against the C-terminal part of MYOM3 showed the presence of two fragments of 110 kDa and 140 kDa in the plasma of *Gaa*^{*-*/-} mice at all ages tested **(****Figure 4****).** These fragments were undetectable in healthy controls (*Gaa*^{+/+} mice). Of note, Gaa-/- mice already display increase of glycogen at these ages.

In conclusion, detection of myomesin or of fragment(s) thereof in a biological fluid seems to be a relevant biomarker to determine whether or not a subject carries muscular glycogenosis, prediction of the development of muscular glycogenosis, screening for a therapeutic agent or technique for muscular glycogenosis, or for the evaluation of the efficacy of a therapeutic agent or technique for muscular glycogenosis.

## Claims

1. A method for diagnosing, for prognosing, for monitoring the evolution, for determining the risk of having or of developing a muscular glycogenosis in a subject, or for determining the efficacy of a treatment of a muscular glycogenosis in a subject, comprising detecting the presence or absence of myomesin protein or of a fragment thereof in a biological fluid sample of said subject.

2. The method according to claim 1, wherein said method comprises :
a) measuring the level of said myomesin protein or of said fragment thereof in said biological fluid sample of the subject; and
b) comparing said level to the level of said myomesin protein or of said fragment thereof in another biological fluid sample collected from said subject or from another subject, and/or to a reference level of said myomesin protein or of said fragment thereof.

3. The method according to claim 1 or 2, wherein said method is for diagnosing a muscular glycogenosis in a subject, comprising :
a) measuring the level of said myomesin protein or of said fragment thereof in a biological fluid sample of the subject; and
b) comparing said level to a reference level of said myomesin protein or of said fragment thereof;
wherein a higher level of said myomesin protein or of said fragment thereof in a biological sample of the subject when compared to the reference level is indicative of a muscular glycogenosis or of a risk of having or of developing a muscular glycogenosis.

4. The method according to claim 1 or 2, wherein said method is for prognosing or for monitoring the evolution of a muscular glycogenosis in a subject in need thereof, comprising:
a) measuring the level of said myomesin protein or of said fragment thereof in a biological fluid sample of the subject,
b) comparing said level to the level of said myomesin protein or of said fragment in a biological fluid sample previously collected in the same subject;
the evolution of the level of said myomesin protein or of said fragment thereof being indicative of the progression of the disease.

5. The method according to claim 1 or 2, wherein said method is for determining the efficacy of a treatment of a muscular glycogenosis in a subject in need thereof, comprising:
a) measuring the level of said myomesin protein or of said fragment thereof in a biological fluid sample of the subject,
b) comparing said level to the level of said myomesin protein or of said fragment in a biological fluid sample previously collected in the same subject;
the evolution of the level of said myomesin protein or of a said fragment thereof being indicative of the progression of the disease and of the efficacy of the treatment.

6. The method according to claim 5, comprising:
a) measuring the level of said myomesin protein or of said fragment thereof in a biological fluid sample of the subject, whereby a reference level is determined; then
b) measuring the level of said myomesin protein or of said fragment in a second biological fluid sample collected in the same subject at a time after administration of the treatment, whereby a test level is determined; and
c) comparing said reference and test levels, the evolution of the level of said myomesin protein or of said fragment thereof being indicative of the progression of the disease and of the efficacy of the treatment.

7. The method according to claim 5 or 6, wherein a reduction of the level of said myomesin protein or of said fragment is indicative of an efficient treatment of the muscular glycogenosis.

8. The method according to any one of claims 1 to 7, wherein said myomesin protein is myomesin 1, myomesin 2 or myomesin 3 or wherein said fragment is a fragment of myomesin 1, myomesin 2 or myomesin 3, wherein said myomesin is preferably myomesin 3 or said fragment is a fragment of myomesin 3, wherein said myomesin is more preferably human myomesin 3 of SEQ ID NO:7 or wherein said fragment is a fragment of human myomesin 3 of SEQ ID NO:7.

9. The method according to claim 8, wherein the fragment of myomesin 3 is a C-terminal fragment.

10. The method according to any one of claims 1 to 9, wherein the biological fluid is blood, serum, plasma, saliva or urine, the biological fluid being preferably plasma or serum.

11. The method according to any one of claims 1 to 10, comprising detecting the presence or absence of said myomesin protein or of said fragment thereof by immunoassay such as enzyme-linked immunosorbent assay (ELISA), lateral flow immunoassay or western-blotting ; by capillary electrophoresis ; or by mass spectrometry, preferably by immunoassay.

12. Use of myomesin protein, in particular of myomesin 3, or of a fragment of myomesin, in particular a fragment of myomesin 3, as a biomarker for diagnosing, prognosing, for monitoring the evolution, or for determining the risk of having or of developing a muscular glycogenosis, or for determining the efficacy of a treatment of a muscular glycogenosis.

13. The method according to any one of claims 1 to 11 or the use of claim 12, wherein said muscular glycogenosis is selected from the group consisting of GSD 0 (muscle glycogen synthase deficiency), GSDII (Pompe disease), GSDIII (glycogen debranching enzyme deficiency), GSDIV (glycogen branching enzyme deficiency), GSDV (myophosphorylase deficiency), GSDVII (phosphofructokinase deficiency), GSDIXd (muscle phosphorylase kinase deficiency), GSDX (muscle phosphoglycerate mutase deficiency), GSDXI (lactate dehydrogenase deficiency), GSDXII (aldolase A deficiency), GSDXIII (β-enolase deficiency), GSDXIV (phosphoglucomutase deficiency, also known as CDG It), and GSDXV (muscle glycogenin deficiency), preferably GSDII (Pompe disease) or GSDIII, more preferably GSDIII.

14. The method according to any one of claims 1 to 11 or the use of claim 12 or 13, wherein said muscular glycogenosis is GSDII (Pompe disease) or GSDIII, more preferably GSDIII.

15. A kit for implementing a method according to any one of claims 1 to 14, comprising the means for detecting said myomesin protein, in particular myomesin 3, or said fragment thereof, in particular a fragment of myomesin 3, and instructions for carrying out said method.
